# EUROPEAN PATENT APPLICATION

(11) **EP 4 806 420 A1**
(43) Date of publication of application: **16.09.2026**
(21) Application number: 25768685.7
(22) Date of filing: 04.03.2025
(51) Int. Cl.: A61K 8/85, A61K 8/37, A61K 8/49, A61K 8/11, A61Q 17/04

(54) **POLYMER-CONTAINING COMPOSITION HAVING INCREASED UV BLOCKING BOOSTING EFFECT AND COSMETIC COMPOSITION COMPRISING SAME**

(30) Priority: 08.03.2024 KR 20240033425; 20.02.2025 KR 20250022366
(71) Applicant: Scai Therapeutics Co., Ltd., Seoul 06524 (KR)
(72) Inventor: KIM, Chul Hwan, Daejeon 35249 (KR); KIM, Kyoung Hee, Daejeon 35249 (KR); PARK, Bum Woo, Cheongju-si, Chungcheongbuk-do 28173 (KR)
(74) Representative: dompatent
(86) International application number: PCT/KR2025/099511
(87) International publication number: WO 2025/188130

(57) **Abstract**

The present disclosure relates to a UV blocking efficacy booster composition comprising a polymer and a cosmetic composition comprising the same. More specifically, by using a polymer as an additive in a cosmetic composition, a sticky feeling of use can be improved, skin penetration can be prevented, and a high SPF (sun protection factor) can be implemented despite a low content of a UV blocking agent, and in vivo SPF can be enhanced.

## Description

### Technical Field

The present disclosure relates to a composition having an enhanced boosting effect on UV blocking, including a polymer. More specifically, the present disclosure relates to a composition having an enhanced boosting effect on UV blocking that can improve the sticky feeling of use, prevents skin penetration, implement a high sun protection factor (SPF) despite a low content of UV blocking agent, and enhance the SPF in a living body by using a polymer as an additive for a cosmetic composition, and a cosmetic composition comprising the same.

### Background Art

UV blocking agents can be broadly classified into inorganic UV blocking agents and organic UV blocking agents. Each UV blocking agent ingredient has a mixing limit set by the Ministry of Food and Drug Safety. Considering the mixing limit, the inorganic UV blocking agent can cause white turbidity on the skin and has the disadvantage of being difficult to apply due to the thick formulation. In addition, the inorganic UV blocking agent is known to cause cancer or increase the risk of dementia when absorbed through the skin. The organic UV blocking agent is also absorbed through the skin, and if the organic UV blocking agent is absorbed into the body at a level exceeding the safety standard, there may be an increased risk of side effects such as cancer or birth defects.

In this way, when the UV blocking agent ingredient is absorbed into the dermis layer of the skin, a side effect that causes skin cancer occurs. To solve this problem, if the UV blocking agent is encapsulated into a material that can enhance SPF, less UV blocking agent ingredient can be used and the phenomenon of absorption into the skin can be suppressed. In addition, the existing sunscreen, which is an organic UV blocking agent, has a high content of organic UV blocking agent at 30% by weight of the total weight of the sunscreen, and accordingly, has the disadvantage of not feeling good when used, due to its sticky properties resulting from the characteristics of the organic UV blocking agent.

Also, recently, there was a problem with false manipulation of SPF among sunscreen products on the market. The common feature of these products was that they used organic UV blocking agent ingredients, and among the organic UV blocking agent ingredients widely used, there were UV-A blocking agent ingredients such as Diethylamino Hydroxybenzoyl Hexyl Benzoate Uvinul A+, UV-B ingredients such as Ethylhexyl Triazone Uvinul T 150, and UV-A blocking agent ingredients such as Bis-Ethylhexyloxyphenol Methoxyphenyl Triazine Tinosorb S, and by using these, it was said that two or three UV blocking agent ingredients were contained.

Normally, it is known that it is not easy to create the effect of SPF 50+ or PA ++++ with these 2 or 3 ingredients when manufacturing sunscreen, and if each ingredient is added at the maximum content, it can cause irritation regardless of the EWG grade, which is problematic. Also, since the SPF value is affected by UV-B blocking, the value increases when a lot of UV-B blocking agent is added, and thus, typically, 5 to 7 % by weight of organic UV blocking agent ingredient is added and a lot of inorganic UV blocking agents related to coverage are added to increase the SPF value. Also, although SPF can be increased by using a UV-A blocking agent, since this requires a large amount of addition, most products use UV-B, and most products use two or three organic UV blocking agent ingredients while mixing inorganic UV blocking agents.

Accordingly, there is a need to develop a UV blocking agent that has the effect of SPF 50+ and PA ++++ while not using an inorganic UV blocking agent and minimizing the content of organic UV blocking agents by including them to less than 5 % by weight, and an additive that can be used in addition to existing UV blocking agents.

### [Prior Art Document]

### [Patent Document]

(Patent Document 1) Korean Laid-open Patent Publication No. 10-2011-0137157 A (2011.12.22)

### SUMMARY OF THE DISCLOSURE

The inventors of the present disclosure, as a result of research to supplement the disadvantages of the prior art as mentioned above, have found that despite having a lower content of UV blocking agent than existing products, it has a booster effect that can maximize UV blocking efficacy, improves the sticky feeling of use, and also suppresses the phenomenon of organic UV blocking agent ingredients being absorbed into the skin, and can minimize environmental pollution because it is naturally decomposed when discarded.

Therefore, it is an object of the present disclosure to provide a UV blocking efficacy booster composition having the functions.

According to one embodiment of the present disclosure, the present disclosure provides a UV blocking efficacy booster composition comprising a polymer, wherein the D₅₀ (volume) of the polymer included in the composition is 0.1 to 10 µm.

Also, according to one embodiment of the present disclosure, the polymer is characterized in that it is a biodegradable polymer.

Also, according to one embodiment of the present disclosure, the polymer is characterized by having at least one functional group of an NH group and a carbonyl group.

Also, according to one embodiment of the present disclosure, the polymer is characterized in that it is at least one selected from the group consisting of poly hydroxyalkanoate (PHA), poly methyl methacrylate (PMMA), poly L-lactic acid (PLLA), and poly ethylene imine (PEI).

According to one embodiment of the present disclosure, the present disclosure provides a cosmetic composition comprising a polymer; and an oil; wherein the D₅₀ (volume) of the polymer contained in the composition is 0.1 to 10 µm.

Also, according to one embodiment of the present disclosure, the present disclosure provides a cosmetic composition characterized in that the polymer encapsulates a UV blocking agent ingredient.

Also, according to one embodiment of the present disclosure, the polymer is characterized in that the composition is contained in an amount of more than 0 and less than or equal to 10 % by weight relative to the total weight of the cosmetic composition.

Also, according to one embodiment of the present disclosure, the cosmetic composition further contains other organic UV blocking agents in addition to the polymer, and the organic UV blocking agent is characterized in that it is at least one selected from the group consisting of ethylhexyl methoxycinnamate (OMC), bis-ethylhexyloxyphenol methoxyphenyl triazine (TS), polysilicon-15, isoamyl p-methoxycinnamate, diethylaminohydroxybenzoylhexyl benzoate, ethylhexyl triazone, butyl methoxydibenzoylmethane, ethylhexyl salicylate, ethylhexyl cinnamate, homosalate, ethylhexyl triazone and octocrylene.

Also, according to one embodiment of the present disclosure, the cosmetic composition is characterized in that it is for UV blocking.

Also, according to one embodiment of the present disclosure, the composition is characterized in that it has a formulation selected from the group consisting of solutions, ointments, external ointments, creams, essences, foams, toners, nourishing toners, softening water, softening toners, packs, fluids, makeup bases, soaps, cleansers, bath preparations, sunscreens, sun oils, suspensions, emulsions, pastes, gels, lotions, powders, cleansing agents containing surfactants, oils, foundations, powder foundations, emulsion foundations, wax foundations, patches, and sprays.

The UV blocking efficacy booster composition according to the present disclosure and the cosmetic composition containing the same have a booster effect capable of maximizing UV blocking efficacy despite containing less UV blocking ingredients than existing products, and improve the feeling of use by containing less UV blocking ingredients that have a sticky feeling of use. In addition, they have a booster effect that can maximize UV blocking efficacy by suppressing the phenomenon of organic UV blocking agent ingredients being absorbed into the skin, and also have the advantage of minimizing environmental pollution as they are naturally decomposed when discarded.

### BRIEF DESCRIPTION OF THE DRAWINGS

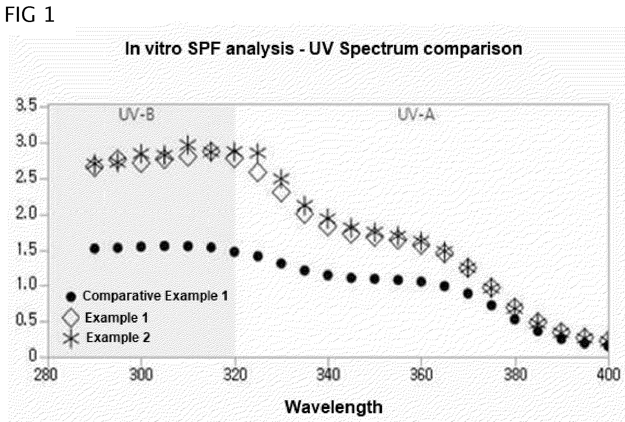

FIG. 1 is a graph of UV spectrum data, which is the result of an in vitro analysis illustrating that the UV-A, B region is broadened overall, and thus, SPF/PA is increased, when the dispersion (including PHA) according to one embodiment of the present disclosure is added.

### DETAILED DESCRIPTION

Hereinafter, the present disclosure will be described in more detail.

The terms and words used in the present specification and claims should not be construed as limited to ordinary or dictionary terms, and should be construed in a sense and concept consistent with the technical idea of the present disclosure, based on the principle that the inventor can properly define the concept of a term to describe his or her invention in the best way possible.

The terminology used herein is only to describe specific embodiments and is not intended to limit the invention. The singular forms "a," "an," and "the" include plural referents unless the context clearly dictates otherwise. It is to be understood that the term "comprise" or "have" as used in the present disclosure is intended to designate the presence of features, numbers, steps, operations, components, parts or combinations thereof described in the specification, but not to preclude the possibility of the presence or addition of one or more other features, numbers, steps, operations, components, parts, or combinations thereof.

First, the present disclosure provides a UV blocking efficacy booster composition comprising a polymer, wherein the D₅₀ (volume) of the polymer contained in the composition is 0.1 to 10 µm.

The inventors of the present disclosure have found that if a polymer is used to suppress the phenomenon of a UV blocking agent ingredient being absorbed into the skin, not only can it have a booster effect that maximizes UV blocking efficacy, but it can also minimize environmental pollution because it is naturally decomposed when discarded.

The UV blocking efficacy booster composition according to the present disclosure comprises a polymer, wherein the polymer may be, but is not limited to, a biodegradable polymer.

The polymer contained in the UV blocking efficacy booster composition according to the present disclosure may be at least one selected from the group consisting of poly hydroxyalkanoate (PHA), poly methyl methacrylate (PMMA), poly L-lactic acid (PLLA), and poly ethylene imine (PEI). When these polymers are used together with a UV blocking agent ingredient, if the phenomenon of the UV blocking agent ingredient being absorbed into the skin is suppressed, it not only has a booster effect that can maximize UV blocking efficacy, but also can minimize environmental pollution because it is naturally decomposed when discarded.

In addition, the polymer contained in the UV blocking efficacy booster composition according to the present disclosure may have at least one functional group of an NH- group and a carbonyl group. When the polymers having such functional groups are used together with a UV blocking agent ingredient, this can suppress the phenomenon of the UV blocking agent ingredient being absorbed into the skin.

In addition, the polymer contained in the UV blocking efficacy booster composition according to the present disclosure may have a D₅₀ (volume) of 0.1 to 10 µm. Here, D₅₀ (volume) means a particle size corresponding to when the percentage of the cumulative particle size distribution in the polymer powder reaches 50% on a volume basis. If the D₅₀ (volume) of the polymer has the above range, when used together with a UV blocking agent ingredient, it has a booster effect that can maximize UV blocking efficacy and can suppress the phenomenon of the UV blocking agent ingredient being absorbed into the skin.

Specifically, the D50 (volume) of the polymer contained in the composition may be 0.1 µm or more, 0.3 µm or more, 0.5 µm or more, 1.0 µm or more, 2.0 µm or more, and may be 10 µm or less, 8 µm or less, 6 µm or less, 5 µm or less, 4 µm or less, 3 µm or less, or 1.5 µm or less.

Next, the present disclosure provides a cosmetic composition comprising the UV blocking efficacy booster composition.

The cosmetic composition of the present disclosure comprises a polymer; and an oil; and is characterized in that the D₅₀ (volume) of the polymer is 0.1 to 10 µm.

The polymer contained in the cosmetic composition of the present disclosure may encapsulate a UV blocking agent ingredient, but is not limited thereto.

The polymer contained in the cosmetic composition of the present disclosure may be contained in an amount of more than 0 and less than or equal to 10 % by weight relative to the total weight of the cosmetic composition, and preferably may be contained in an amount of 0.001 % by weight or more, 0.01 % by weight or more, 0.1 % by weight or more, 1.0 % by weight or more, 2.0 % by weight or more, and may be contained in an amount of 8 % by weight or less, 6 % by weight or less, 5 % by weight or less, 4 % by weight or less, or 3 % by weight or less.

If the content of the polymer is higher than the above range, there is a problem that a phenomenon of being pushed on the skin occurs.

The cosmetic composition of the present disclosure may further contain other organic UV blocking agents in addition to the polymer. The organic UV blocking agent contained in the cosmetic composition of the present disclosure may be contained in an amount of 3 to 10% by weight relative to the total weight of the cosmetic composition. The cosmetic composition of the present disclosure has a booster effect capable of maximizing UV blocking efficacy despite a small content of the organic UV blocking agent by containing the polymer as described above.

If the content of the organic UV blocking agents is lower than 3% by weight, there is a problem that the UV blocking effect is insufficient, and if the content of the organic UV blocking agents is higher than 10% by weight, there is a problem of poor feeling of use (stickiness).

Specifically, the organic UV blocking agent may be contained in an amount of 3.0% by weight or more, 3.5% by weight or more, 4.0% by weight or more, 4.5% by weight or more, and may be contained in an amount of 10.0% by weight or less, 8.0% by weight or less, 6.0% by weight or less, or 4.5% by weight or less, relative to the total weight of the cosmetic composition.

Specifically, the organic UV blocking agent may be at least one selected from the group consisting of ethylhexyl methoxycinnamate (OMC), bis-ethylhexyloxyphenol methoxyphenyl triazine (TS), polysilicon-15, isoamyl p-methoxycinnamate, diethylaminohydroxybenzoyl hexyl benzoate, ethylhexyl triazone, butyl methoxydibenzoylmethane, ethylhexyl salicylate, ethylhexyl cinnamate, homosalate, ethylhexyl triazone, and octocrylene.

The cosmetic composition of the present disclosure comprises a UV blocking efficacy booster composition, and in this case, if bis-ethylhexyloxyphenol methoxyphenyl triazine (TS) is used as an organic UV blocking agent, it can have a synergistic effect on UV blocking that is 5 times or more, or 10 times or more, compared to when it is not used.

In addition, the cosmetic composition of the present disclosure comprises a UV blocking efficacy booster composition, and in this case, if ethylhexyl methoxycinnamate (OMC) is used as an organic UV blocking agent, it can have a synergistic effect on UV blocking that is 1.5 times or more, 2 times or more, 5 times or more, or 10 times or more, compared to when it is not used.

The specific examples of the polymer are identical to the contents of the polymer contained in the UV blocking efficacy booster composition discussed above.

In addition, the polymer may be contained as an additive in an existing UV blocking agent.

The existing UV blocking agent can be any commercially available UV blocking agent.

The cosmetic composition of the present disclosure can be used as an additive to all commercially available UV blocking agents. When used as an additive, it can be confirmed through Experimental Example 4 described below that the peak wide of the ultraviolet spectrum and the SPF/PA value are increased. In addition, even if the present disclosure is used as an additive at less than 1%, a boosting effect, which is an effect of the present disclosure, occurs.

The cosmetic composition of the present disclosure may contain an inorganic UV blocking agent, and the inorganic UV blocking agent may be contained in an amount of 1 to 10% by weight relative to the total weight of the cosmetic composition.

If the content of the inorganic UV blocking agent is lower than 1% by weight, there is a problem that the physical UV blocking effect is insufficient, and if the content of the inorganic UV blocking agent is higher than 10% by weight, there is a problem that the phenomenon of white turbidity of the skin occurs.

Specifically, the inorganic UV blocking agent may be contained in an amount of 1.0% by weight or more, 2.0% by weight or more, 3.0% by weight or more, 4.0% by weight or more, and may be contained in an amount of 10.0% by weight or less, 8.0% by weight or less, 6.0% by weight or less, or 4.0% by weight or less, relative to the total weight of the cosmetic composition.

Specifically, the inorganic UV blocking agent may be at least one selected from the group consisting of zinc oxide, titanium dioxide, and cerium oxide.

The oil contained in the cosmetic composition of the present disclosure can be used without particular limitation as long as it is used in the cosmetic composition, and for example, MCT oil, etc. can be used.

The cosmetic composition of the present disclosure has a formulation selected from the group consisting of solutions, ointments, external ointments, creams, essences, foams, toners, nourishing toners, softening water, softening toners, packs, fluids, makeup bases, soaps, cleansers, bath preparations, sunscreens, sun oils, suspensions, emulsions, pastes, gels, lotions, powders, cleansing agents containing surfactants, oils, foundations, powder foundations, emulsion foundations, wax foundations, patches, and sprays.

The method for preparing the cosmetic composition of the present disclosure can be used without particular limitation as long as it is a device capable of applying shear stress, and specifically, one or more devices selected from the group consisting of a homogenizer, a high-pressure homogenizer, a packed silica, a rotor-stator homogenizer, a piston homogenizer, and a tube homogenizer can be used.

Specifically, the polymer of the present disclosure can be introduced into the devices together with an emulsifier, and then mixed.

As the emulsifier, polyoxyethylene sorbitan (Tween), polyoxyethylene-polyoxypropylene copolymer (poloxamer), sorbitan ester (Span), polyoxyethylene ether (Brij), and glycerin stearate citrate can be used, and an emulsifier such as Tween can be preferably used.

In addition, the mixing can be performed at a speed of 5000 to 10000 RPM for 3 to 10 minutes.

Thereafter, the mixing can be carried out 1 to 5 times at a pressure of 1000 to 2000 bar using a high-pressure homogenizer (HPH).

Thereafter, after adding distilled water again, the mixing can be carried out at a speed of 2000 to 5000 RPM for 5 to 15 minutes, and then the high-pressure homogenizer can be used again in the same way as above.

Thereafter, the solvent, such as chloroform, can be removed using a speed var concentrator to prepare the final dispersion, which is a UV blocking efficacy booster composition.

If necessary, an organic UV blocking agent such as TS or OMC can be added in advance during the step of preparing the booster composition.

Thereafter, various ingredients used in cosmetic compositions, such as MCT oil, phytosphingosine, lactic acid, 1-2-hexanediol, etc., and various organic or inorganic UV blocking agent ingredients discussed above can be mixed with the dispersion to prepare a cosmetic composition.

The present disclosure will be described in more detail with reference to the following preferred examples. However, the scope of protection of the present disclosure is not limited by these examples.

### [Examples]

### 1. Chemicals used

### 1) Preparation of dispersion

Chloroform, glycerin stearate citrate (GSC), UV-filter substances, for example, bis-ethylhexyloxyphenol methoxyphenyl triazine (TS, TINOSORB S), ethylhexyl methoxycinnamate (OMC); polyhydroxyalkanoate (PHA), 1-2-hexanediol (Product name: Activonol-6)

### 2) Preparation of cosmetic composition

Caprylic/capric triglyceride (MCT Oil), Phytosphingosine, Lactic acid, Titanium dioxide (e.g., TiO₂ dispersion (aqueous, 30%), a commercially available product from Agarbatti), 1-2-Hexanediol (Activonol-6)

### Devices used

### 1) Preparation of dispersion

Homomixer, High-pressure homogenizer (PandaPLUS 2000 from GEA Niro Soavi company), Rotary evaporator, Dryer (Oven), Particle size analyzer (MasterSizer3000 from Malvern company)

### 2) Preparation of cosmetic composition

Homomixer, high-pressure homogenizer (PandaPLUS 2000 from GEA Niro Soavi company)

### 3) Analysis of sun protection factor (SPF)

In vitro SPF measurement (SPF-290AS_Solar Light company, owned by Korea Ceramic Technology Institute)

In vivo SPF measurement (External analysis agency_Korea Dermatological Research Institute at Gyeonggi-do, Korea/Skinmed at Daejeon, Korea)

### 1. Preparation of dispersion

### Dispersion Preparation Example 1. Preparation of polyhydroxyalkanoate (PHA) dispersion solution

2.8 g of glycerin stearate citrate (GSC, dermofeel^{®} GSC SG from EVONIK company) powder was added to a beaker and 14 g of polyhydroxyalkanoate (PHA, raw material from CJ-bio company) powder was added. Thereafter, chloroform was added so that the total weight became 400 g, and the homomixer was operated at 7,000 RPM for 5 minutes.

The solution thus prepared was subjected to a high-pressure homogenizer (HPH) to perform the process three times at a pressure of 1,300 bar, thereby preparing a final solution.

Thereafter, 700 g of distilled water was added to the beaker, and the homomixer was operated at 3,000 RPM, and 300 g of the previously prepared solution was slowly added, and then the mixture was dispersed by operating at 5,000 RPM for 10 minutes, and then the HPH was used to perform the process 5 times at a pressure of 1,300 bar.

Thereafter, a rotary evaporator was used to completely remove chloroform at a temperature of 60°C, and a speed var concentrator was used to additionally remove some distilled water to prepare a final dispersion.

The dry solid content of the prepared dispersion (after drying in a drying oven at 80°C for 12 hours or more, the weight is measured to calculate the content of the residue) was confirmed to be 21% by weight as a result of analysis, and the particle size distribution was confirmed to be D₅₀ (volume) 0.6 µm as a result of analysis using the MasterSizer 3000 equipment of Malvern company.

### Dispersion Preparation Example 2. Preparation of PHA dispersion solution containing organic UV blocking agent

2.92 g of glycerin stearate citrate (GSC, dermofeel^{®} GSC SG from EVONIK company) powder and 14.60 g of polyhydroxyalkanoate (PHA) powder were added to the beaker.

Thereafter, chloroform was added so that the total weight became 400 g, and 5.26 g of bis-ethylhexyloxyphenol methoxyphenyl triazine (TS, TINOSORB S) powder and 12.26 g of ethylhexyl methoxycinnamate (OMC) solution were additionally added, and then the homomixer was operated at 7,000 RPM for 5 minutes.

The solution thus prepared was subjected to HPH to perform the process three times at a pressure of 1,300 bar, thereby preparing a final solution.

Thereafter, 700 g of distilled water was added to the beaker, and the homomixer was operated at 3,000 RPM, and 300 g of the previously prepared solution was slowly added, and then the mixture was dispersed by operating at 5,000 RPM for 10 minutes, and then the HPH was used to perform the process 5 times at a pressure of 1,300 bar.

Thereafter, a rotary evaporator was used to completely remove chloroform at a temperature of 60°C, and to additionally remove some distilled water, thereby preparing a final dispersion.

The dry solid content of the prepared dispersion was confirmed to be 51% by weight as a result of analysis, and the particle size distribution was confirmed to be D₅₀ (volume) 0.7 µm as a result of analysis.

### Dispersion Preparation Example 3. Preparation of polymethylmethacrylate (PMMA) dispersion solution

A dispersion was prepared in the same manner as in the Dispersion Preparation Example 1, except that in the Dispersion Preparation Example 1, 14 g of polymethylmethacrylate (PMMA, molecular weight 350k, a product from Aldrich company) was used instead of polyhydroxyalkanoate.

The dry solid content of the prepared dispersion was confirmed to be 18% by weight as a result of analysis, and the particle size distribution was confirmed to be D₅₀ (volume) 0.6 µm as a result of analysis.

### Dispersion Preparation Example 4. Preparation of poly L-lactic acid (PLLA) dispersion solution

A dispersion was prepared in the same manner as in the Dispersion Preparation Example 1, except that in the Dispersion Preparation Example 1, 14 g of poly L-lactic acid (PLLA, molecular weight 100k, a product from Aldrich company) was used instead of polyhydroxyalkanoate.

The dry solid content of the prepared dispersion was confirmed to be 18% by weight as a result of analysis, and the particle size distribution was confirmed to be D₅₀ (volume) 1.2 *µ*m as a result of analysis.

### Dispersion Preparation Example 5. Preparation of polystyrene dispersion solution

A dispersion was prepared in the same manner as in the Dispersion Preparation Example 1, except that in the Dispersion Preparation Example 1, 14 g of polystyrene (PS, molecular weight 350k, a product from Aldrich company) was used instead of polyhydroxyalkanoate.

The dry solid content of the prepared dispersion was confirmed to be 18% by weight as a result of analysis, and the particle size distribution was confirmed to be D₅₀ (volume) 0.6 *µ*m as a result of analysis.

The polystyrene has a structure that does not have an NH-group and a carbonyl group, unlike the polymers of the present disclosure, and was therefore used to compare the effects of the present disclosure.

### Dispersion Preparation Example 6. Preparation of PHA dispersion solution containing organic UV blocking agent

A dispersion was prepared in the same manner as in the Dispersion Preparation Example 2, except that in the Dispersion Preparation Example 2, ethylhexyl methoxycinnamate (OMC) was not used, and 17.52 g of ethylhexyloxyphenol methoxyphenyl triazine (TS, TINOSORB S) powder was used instead of OMC.

The dry solid content of the prepared dispersion was confirmed to be 38% by weight as a result of analysis, and the particle size distribution was confirmed to be D₅₀ (volume) 0.7 µm as a result of analysis.

### 2. Preparation of cosmetic composition

### Example 1.

28.6 % by weight of the dispersion prepared in the Dispersion Preparation Example 1, 3.3 % by weight of titanium dioxide (TiO₂, TiO₂ dispersion (water-soluble, 30%), a product from Agarbatti company), 44.0 % by weight of caprylic/capric triglyceride (MCT Oil), 2.0 % by weight of phytosphingosine (PhSG, a product from Phytosphingosine Solus Biotech company), 4.5 % by weight of 1 mol lactic acid, 2.0 % by weight of 1-2-hexanediol (Activonol-6), 1.8 % by weight of bis-ethylhexyloxyphenol methoxyphenyl triazine (TS, TINOSORB S), 4.2 % by weight of ethylhexyl methoxycinnamate (OMC), and the remainder of distilled water were mixed to prepare a cosmetic composition.

### Example 2. Preparation of cosmetic composition

23.5 % by weight of the dispersion prepared in the Dispersion Preparation Example 2, 3.3 % by weight of titanium dioxide (TiO₂, TiO₂ dispersion (water-soluble, 30%), a product from Agarbatti company (manufactured by SINA BT Co., Ltd.)), 44.0 % by weight of caprylic/capric triglyceride (MCT Oil), 2.0 % by weight of phytosphingosine, 4.5 % by weight of 1 mol lactic acid, 2.0 % by weight of 1-2-hexanediol (Activonol-6), and the remainder of distilled water were mixed to prepare a cosmetic composition.

### Example 3. Preparation of cosmetic composition

A cosmetic composition was prepared in the same manner as in the Example 2, except that the dispersion prepared in the Dispersion Preparation Example 2 was used in an amount of 35.3% by weight and titanium dioxide was used in an amount of 5.0% by weight.

### Example 4. Preparation of cosmetic composition

A cosmetic was prepared in the same manner as in the Example 1, except that bis-ethylhexyloxyphenol methoxyphenyl triazine (TS, TINOSORB S) was used in an amount of 0.9% by weight and ethylhexyl methoxycinnamate (OMC) was used in an amount of 2.1% by weight.

### Example 5. Preparation of cosmetic composition

A cosmetic was prepared in the same manner as in the Example 4, except that 33.3% by weight of the dispersion prepared in Dispersion Preparation Example 3 was used.

### Example 6. Preparation of cosmetic composition

A cosmetic was prepared in the same manner as in the Example 4, except that 33.3% by weight of the dispersion prepared in Dispersion Preparation Example 4 was used.

### Example 7. Preparation of cosmetic composition

A cosmetic was prepared in the same manner as in the Example 4, except that 33.3% by weight of the dispersion prepared in Dispersion Preparation Example 5 was used.

### Example 8. Preparation of cosmetic composition

A cosmetic was prepared in the same manner as in the Example 1, except that 28.6% by weight of the dispersion prepared in Dispersion Preparation Example 1 was used, and 3.0% by weight of bis-ethylhexyloxyphenol methoxyphenyl triazine (TS, TINOSORB S) was used instead of bis-ethylhexyloxyphenol methoxyphenyl triazine (TS, TINOSORB S) and ethylhexyl methoxycinnamate (OMC).

### Example 9. Preparation of cosmetic composition

A cosmetic was prepared in the same manner as in Example 8, except that 3.0 % by weight of ethylhexyl methoxycinnamate (OMC) was used instead of bis-ethylhexyloxyphenol methoxyphenyl triazine (TS, TINOSORB S).

### Example 10. Preparation of cosmetic composition

A cosmetic was prepared in the same manner as in Example 8, except that 3.0 % by weight of methylenebis-benzotriazolyltetramethylbutylphenol (TM) was used instead of bis-ethylhexyloxyphenol methoxyphenyl triazine (TS, TINOSORB S).

### Example 11. Preparation of cosmetic composition

A cosmetic was prepared in the same manner as in Example 8, except that 3.0% by weight of isoamyl p-methoxycinnamate (IMC) was used instead of bis-ethylhexyloxyphenol methoxyphenyl triazine (TS, TINOSORB S).

### Example 12. Preparation of cosmetic composition

A cosmetic was prepared in the same manner as in Example 8, except that 3.0% by weight of homosalate (Parsol HMS) was used instead of bis-ethylhexyloxyphenol methoxyphenyl triazine (TS, TINOSORB S).

### Example 13. Preparation of cosmetic composition

A cosmetic composition was prepared by adding 2.0% by weight of the dispersion prepared in the Dispersion Preparation Example 1 instead of water to a commercially available product, Celenin the young UV Moisture Milk.

### Example 14. Preparation of cosmetic composition

A cosmetic composition was prepared by adding 11.4% by weight of the dispersion prepared in the Dispersion Preparation Example 6 instead of water and bis-ethylhexyloxyphenol methoxyphenyl triazine (TS, TINOSORB S) to a commercially available product, Celenin the young UV Moisture Milk.

### Comparative Example 1. Preparation of cosmetic composition

A cosmetic was prepared in the same manner as in the Example 1, except that the dispersion prepared in Dispersion Preparation Example 1 was not used.

### Comparative Example 2. Preparation of cosmetic composition

A cosmetic was prepared in the same manner as in the Comparative Example 1, except that bis-ethylhexyloxyphenol methoxyphenyl triazine (TS, TINOSORB S) was used in an amount of 0.9% by weight and ethylhexyl methoxycinnamate (OMC) was used in an amount of 2.1% by weight.

### Comparative Example 3. Preparation of cosmetic composition

A cosmetic was prepared in the same manner as in the Example 8, except that in the Example 8, no dispersion was used.

### Comparative Example 4. Preparation of cosmetic composition

A cosmetic was prepared in the same manner as in the Example 9, except that in the Example 9, no dispersion was used.

### Comparative Example 5. Preparation of cosmetic composition

A cosmetic was prepared in the same manner as in the Example 10, except that in the Example 10, no dispersion was used.

### Comparative Example 6. Preparation of cosmetic composition

A cosmetic was prepared in the same manner as in the Example 11, except that in the Example 11, no dispersion was used.

### Comparative Example 7. Preparation of cosmetic composition

A cosmetic was prepared in the same manner as in the Example 12, except that in the Example 12, no dispersion was used.

### Comparative Example 8. Preparation of cosmetic composition

A commercial product, Celenin the young UV Moisture Milk, was used.

### Experimental Examples

### Experimental Example 1. In vitro SPF and in vivo SPF/PA measurements (Comparative Example 1, Examples 1 to 3)

### In vitro SPF measurement method

Cosmetic formulations were applied to each of five polymethylmethacrylate (PMMA) plates in an amount of 1.3 mg/cm²; Measured at 9 points per plate; SPF-290AS from Solar Light company, using equipment owned by Korea Ceramic Technology Institute.

### In vivo SPF/PA measurement method

The test was conducted by an external testing agency (Korea Institute of Dermatological Sciences, Seoul, Korea / Skinmed Clinical Trial Center Co., Ltd., Daejeon, Korea) according to the standard sun protection factor measurement method (ISO24444:2019/ISO24442:2011), and the results are shown in Table 1.

**Table 1:**

| | Composition | INCI name | Comparative Example 1 | Example 1 | Example 2 | Example 3 |
|---|---|---|---|---|---|---|
| | | | % by weight | % by weight | % by weight | % by weight |
| A | MCT Oil | caprylic/capric triglyceride | 44.0 | 44.0 | 44.0 | 44.0 |
| A | PhSG | phytosphingosine | 2.0 | 2.0 | 2.0 | 2.0 |
| A | Activonol-6 | 1,2-hexanediol | 2.0 | 2.0 | 2.0 | 2.0 |
| A | Lactic acid (1 mol) | lactic acid | 4.5 | 4.5 | 4.5 | 4.5 |
| A | TS | bis-ethylhexyloxyphenol methoxyphenyl triazine | 1.8 | 1.8 | | |
| A | OMC | ethylhexyl methoxycinnamate | 4.2 | 4.2 | | |
| B | TiO2 Dispersion (30%) | titanium dioxide; water | 1.0 | 1.0 | 1.0 | 1.5 |
| | | | 2.3 | 2.3 | 2.3 | 3.5 |
| B | Dispersion Preparation Example 1 | Polyhydroxyalkanoate;glycerin stearate citrate; water | | 5.0 | | |
| | | | | 1.0 | | |
| | | | | 22.6 | | |
| B | Dispersion Preparation Example 2 | Polyhydroxyalkanoate; glycerin stearate citrate; bis-ethylhexyloxyphenol methoxyphenyl triazine; ethylhexyl methoxycinnamate; water | | | 5.0 | 7.5 |
| | | | | | 1.0 | 1.5 |
| | | | | | 1.8 | 2.7 |
| | | | | | 4.2 | 6.3 |
| | | | | | 11.5 | 17.3 |
| B | Distilled water | water | added up to 100 | added up to 100 | added up to 100 | added up to 100 |
| In vitro SPF | | | 26 | 120 | 118 | 133 |
| In vivo SPF | | | 15 | 20 | 44 | 52 |
| In vivo PA | | | PA++ | PA+++ | PA+++ | PA++++ |

As shown in the results in the Table 1, it was confirmed that in vitro SPF significantly increased in Examples 1 to 3 compared to Comparative Example 1 that did not contain PHA.

When comparing Comparative Example 1 and Example 1, it was confirmed that SPF increased slightly and PA increased significantly, and when comparing Comparative Example 1 and Example 2, it was confirmed that both SPF/PA of Example 3 significantly increased as UV blocking ingredients such as TS or OMC were encapsulated into PHA. This can be attributed to the fact that the efficiency increased as the organic UV blocking ingredients were encapsulated into PHA and were not absorbed into the skin.

In addition, as shown in Example 3, since SPF 50+/PA++++ is achieved even with a small amount of inorganic UV blocking agent and organic UV blocking agent, it can be confirmed that the effect of enhancing the in vivo sun protection factor is significant when the dispersion presented in the present disclosure is comprised.

### Experimental Example 2. In vitro SPF and in vivo SPF/PA measurements (Comparative Example 2, Examples 4 to 7)

### In vitro SPF measurement method

Cosmetic formulations were applied to each of five polymethylmethacrylate (PMMA) plates in an amount of 1.3 mg/cm²; Measured at 9 points per plate; SPF-290AS from Solar Light company, using equipment owned by Korea Ceramic Technology Institute.

**Table 2**

| | Composition | INCI name | Comparative Example 2 | Example 4 | Example 5 | Example 6 | Example 7 |
|---|---|---|---|---|---|---|---|
| | | | % by weight | % by weight | % by weight | % by weight | % by weight |
| A | MCT Oil | caprylic/capric triglyceride | 44.0 | 44.0 | 44.0 | 44.0 | 44.0 |
| A | PhSG | phytosphingosine | 2.0 | 2.0 | 2.0 | 2.0 | 2.0 |
| A | Activonol-6 | 1,2-hexanediol | 2.0 | 2.0 | 2.0 | 2.0 | 2.0 |
| A | Lactic acid (1mol) | lactic acid | 4.5 | 4.5 | 4.5 | 4.5 | 4.5 |
| A | TS | bis-ethylhexyloxyphenol methoxyphenyl triazine | 0.9 | 0.9 | 0.9 | 0.9 | 0.9 |
| A | OMC | ethylhexyl methoxycinnamate | 2.1 | 2.1 | 2.1 | 2.1 | 2.1 |
| B | TiO2 Dispersion (30%) | titanium dioxide; | 1.0 | 1.0 | 1.0 | 1.0 | 1.0 |
| | | water | 2.3 | 2.3 | 2.3 | 2.3 | 2.3 |
| B | Dispersion Preparation Example 1 | polyhydroxyalkanoate; | | 5.0 | | | |
| | | glycerin stearate citrate; | | 1.0 | | | |
| | | water | | 22.6 | | | |
| B | Dispersion Preparation Example 3 | polymethyl methacrylate; | | | 5.0 | | |
| | | glycerin stearate citrate; | | | 1.0 | | |
| | | water | | | 27.3 | | |
| B | Dispersion Preparation Example 4 | polylactic acid; | | | | 5.0 | |
| | | glycerin stearate citrate; | | | | 1.0 | |
| | | water | | | | 27.3 | |
| B | Dispersion Preparation Example 5 | polystyrene; | | | | | 5.0 |
| | | glycerin stearate citrate; | | | | | 1.0 |
| | | water | | | | | 27.3 |
| B | Distilled water | water | added up to 100 | added up to 100 | added up to 100 | added up to 100 | added up to 100 |
| In vitro SPF | | | 12 | 47 | 41 | 46 | 15 |

As shown in the results in the Table 2, it was confirmed that the in vitro SPF significantly increased in Example 4 compared to Comparative Example 2 that did not contain PHA. In addition, it was confirmed that the SPF significantly increased even in Examples 4 and 5, which contained PMMA and PLLA instead of PHA, as compared to Comparative Example 2.

However, in the case of Example 7, which used PS without NH- or carbonyl functional groups instead of PHA, since in vitro SPF did not increase as compared to Comparative Example 2, it can be seen that the resin additive to be used should contain a functional group to significantly enhance in vivo sun protection factor. The molecular structures of the resin additives used in this test are presented in Table 3 below.

**Table 3:**

| Types of resin | INCI name | Molecular structure |
|---|---|---|
| PHA(Poly Hydroxy Alkanoate) | polyhydroxyalkanoate | 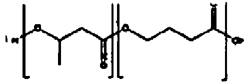 |
| PMMA(Poly Methyl MethAcrylate) | polymethyl methacrylate | 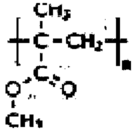 |
| PLLA(Poly L-Lactic Acid) | polylactic acid | 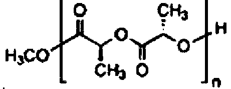 |
| PS(Poly Styrene) | polystyrene | 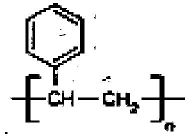 |
| PEI(Poly Ethylen Imine) | polyethyleneimine | 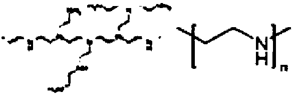 |

### Experimental Example 3. Comparison of the sun protection factor enhancement effect of PHA Dispersion

### In vitro SPF measurement method

Cosmetic formulations were applied to each of five polymethylmethacrylate (PMMA) plates in an amount of 1.3 mg/cm²; Measured at 9 points per plate; SPF-290AS from Solar Light company, using equipment owned by Korea Ceramic Technology Institute.

**Table 4:**

| | Composition | INCI name | Comparat ive Example 3 (Placebo) | Example 8 (Sample) | Comparat ive Example 4 (Placebo) | Example 9 (Sample) | Comparat ive Example 5 (Placebo) | Example 10 (Sample) |
|---|---|---|---|---|---|---|---|---|
| | | | % by weight | % by weight | % by weight | % by weight | % by weight | % by weight |
| A | MCT Oil | caprylic/capric triglyceride | 44.0 | 44.0 | 44.0 | 44.0 | 44.0 | 44.0 |
| A | PhSG | Phytosphingosine | 2.0 | 2.0 | 2.0 | 2.0 | 2.0 | 2.0 |
| A | Activonol-6 | 1,2-hexanediol | 2.0 | 2.0 | 2.0 | 2.0 | 2.0 | 2.0 |
| A | Lactic acid (1 mol) | lactic acid | 4.5 | 4.5 | 4.5 | 4.5 | 4.5 | 4.5 |
| A | TS | bis-ethylhexyloxyphenol methoxyphenyl triazine | 3.0 | 3.0 | | | | |
| A | OMC | ethylhexyl methoxycinnamate | | | 3.0 | 3.0 | | |
| A | TM | methylene bis-benzotriazolyl tetramethylbutylphenol | | | | | 3.0 | 3.0 |
| B | TiO2 dispersion (30%) | titanium dioxide; | 1.0 | 1.0 | 1.0 | 1.0 | 1.0 | 1.0 |
| | | water | 2.3 | 2.3 | 2.3 | 2.3 | 2.3 | 2.3 |
| B | Dispersion Preparation Example 1 | polyhydroxyalkanoate; | | 5.0 | | 5.0 | | 5.0 |
| | | glycerin stearate citrate; | | 1.0 | | 1.0 | | 1.0 |
| | | water | | 22.6 | | 22.6 | | 22.6 |
| B | Distilled water | water | added up to 100 | added up to 100 | added up to 100 | added up to 100 | added up to 100 | added up to 100 |
| In vitro SPF | | | 10 | 62 | 8 | 23 | 3 | 5 |
| SPF increase/decrease rate * | | | | +520% | | +188% | | +67% |
| * (Sample value - Placebo value)/Placebo value X 100% | | | | | | | | |

**Table 5:**

| | Composition | INCI name | Comparat ive Example 6 (Placebo) | Example 11 (Sample) | Comparat ive Example 7 (Placebo) | Example 12 (Sample) |
|---|---|---|---|---|---|---|
| | | | % by weight | % by weight | % by weight | % by weight |
| A | MCT Oil | caprylic/capric triglyceride | 44.0 | 44.0 | 44.0 | 44.0 |
| A | PhSG | phytosphingosine | 2.0 | 2.0 | 2.0 | 2.0 |
| A | Activonol-6 | 1,2-hexanediol | 2.0 | 2.0 | 2.0 | 2.0 |
| A | Lactic acid (1 mol) | lactic acid | 4.5 | 4.5 | 4.5 | 4.5 |
| A | IMC | isoamyl p-methoxycinnamate | 3.0 | 3.0 | | |
| A | Parsol HMS | homosalate | | | 3.0 | 3.0 |
| B | TiO2 dispersion (30%) | titanium dioxide; | 1.0 | 1.0 | 1.0 | 1.0 |
| | | water | 2.3 | 2.3 | 2.3 | 2.3 |
| B | Dispersion preparation example 1 | polyhydroxyalkanoate; | | 5.0 | | 5.0 |
| | | glycerin stearate citrate; | | 1.0 | | 1.0 |
| | | water | | 22.6 | | 22.6 |
| B | Distilled water | water | added up to 100 | added up to 100 | added up to 100 | added up to 100 |
| In vitro SPF | | 9 | 22 | 3 | 5 | |
| SPF increase/decrease rate * | | | +144% | | +67% | |
| * (Sample value - Placebo value)/Placebo value X 100% | | | | | | |

As shown in the results in the Tables 4 and 5, when comparing the amount of change in SPF when PHA dispersion was added (Sample, Example) and when it was not added (Placebo, Comparative Example), depending on the type of organic UV blocking agents, it can be seen that the effect of enhancing in vivo sun protection factor varies depending on the type of organic UV blocking agents, and it was confirmed that among them, Example 8 containing TS organic UV blocking agent showed the most significant effect.

In addition, although the sun protection factor was enhanced in Example 10 containing TM organic UV blocking agent and Example 12 containing Parsol HMS organic UV blocking agent, it could not be considered to have a significant effect because the SPF value was low.

Therefore, when examining the sun protection factor enhancement effect of PHA dispersion, it was found that the effect was in the order of TS ≫ OMC > IMC > TM = Parsol HMS.

### Experimental Example 4. Comparison of sun protection factors measured by using the composition of the present disclosure as an additive to existing commercial UV blocking agents

### In vitro SPF measurement method

Cosmetic formulations were applied to each of five polymethylmethacrylate (PMMA) plates in an amount of 1.3 mg/cm²; Measured at 9 points per plate; SPF-290AS from Solar Light company, using equipment owned by Korea Ceramic Technology Institute.

**Table 6:**

| | Composition | INCI name | Comparative Example 8 | Example 13 | Example 14 |
|---|---|---|---|---|---|
| | | | % by weight | % by weight | % by weight |
| A | CELENIN THE YOUNG UV Moisture Milk | | | | |
| A | TS | bis-ethylhexyloxyphenol methoxyphenyl triazine | 2.20 | 2.20 | |
| B | Dispersion preparation example 1 | Polyhydroxyalkanoate; | | 0.41 | |
| | | glycerin stearate citrate; | | 0.08 | |
| | | water | | 1.51 | |
| B | Dispersion preparation example 6 | Polyhydroxyalkanoate; | | | 1.84 |
| | | glycerin stearate citrate; | | | 0.36 |
| | | bis-ethylhexyloxyphenol methoxyphenyl triazine; water | | | 2.20 |
| | | | | | 7.00 |
| In vitro SPF | | | 42 | 147 | 375 |
| In vivo SPF | | | 40 | 50 | 55 |
| In vivo PA | | | PA++ | PA++++ | PA++++ |

As shown in the results in the Table 6, it was confirmed that when the dispersion suggested in the present disclosure was added to Celenin the young UV Moisture Milk, a commercial product containing various organic UV blocking agents, both the in vitro SPF and in vivo SPF/PA significantly increased.

In addition, when changed to the dispersion in which PHA internally encapsulates TS which is an organic UV blocking ingredient contained in the Celenin the young UV Moisture Milk, a commercial product, since the in vivo SPF significantly increased to 55, it can be seen that the UV blocking efficiency is further increased as explained in Experimental Example 1.

In addition, since the SPF/PA is increased by adding only a small amount of the dispersion (Dispersion Preparation Example 1) presented in this patent to a commercial product, it is expected to be universally applicable as a UV blocking booster when used as an additive to other sunscreen product groups.

## Claims

1. A UV blocking efficacy booster composition containing a polymer, wherein D₅₀ (volume) of the polymer contained in the composition is 0.1 to 10 µm.

2. The UV blocking efficacy booster composition according to claim 1, wherein the polymer is a biodegradable polymer.

3. The UV blocking efficacy booster composition according to claim 1, wherein the polymer has at least one functional group of an NH-group and a carbonyl group.

4. The UV blocking efficacy booster composition according to claim 1, wherein the polymer is at least one selected from the group consisting of poly hydroxyalkanoate (PHA), poly methyl methacrylate (PMMA), poly L-lactic acid (PLLA), and poly ethylene imine (PEI).

5. A cosmetic composition containing a polymer; and an oil, wherein the D₅₀ (volume) of the polymer is 0.1 to 10 µm.

6. The cosmetic composition according to claim 5, wherein the polymer encapsulates a UV blocking agent ingredient.

7. The cosmetic composition according to claim 5, wherein the polymer is contained in an amount of more than 0 and less than or equal to 10% by weight relative to the total weight of the cosmetic composition.

8. The cosmetic composition according to claim 5, wherein the polymer is a biodegradable polymer.

9. The cosmetic composition according to claim 5, wherein the polymer has at least one functional group of an NH-group and a carbonyl group.

10. The cosmetic composition according to claim 5, wherein the polymer is at least one selected from the group consisting of poly hydroxyalkanoate (PHA), poly methyl methacrylate (PMMA), poly L-lactic acid (PLLA), and poly ethylene imine (PEI).

11. The cosmetic composition according to claim 5, wherein the cosmetic composition further comprises another organic UV blocking agent other than the polymer, and the organic UV blocking agent is at least one selected from the group consisting of ethylhexyl methoxycinnamate (OMC), bis-ethylhexyloxyphenol methoxyphenyl triazine (TS), polysilicon-15, isoamyl p-methoxycinnamate, diethylaminohydroxybenzoyl hexyl benzoate, ethylhexyl triazone, butyl methoxydibenzoylmethane, ethylhexyl salicylate, ethylhexyl cinnamate, homosalate, ethylhexyl triazone, and octocrylene.

12. The cosmetic composition according to claim 5, wherein the cosmetic composition is for UV blocking.

13. The cosmetic composition according to claim 5, wherein the composition has a formulation selected from the group consisting of solutions, ointments, external ointments, creams, essences, foams, toners, nourishing toners, softening water, softening toners, packs, fluids, makeup bases, soaps, cleansers, bath preparations, sunscreens, sun oils, suspensions, emulsions, pastes, gels, lotions, powders, cleansing agents containing surfactants, oils, foundations, powder foundations, emulsion foundations, wax foundations, patches, and sprays.
